# EUROPEAN PATENT SPECIFICATION

(11) **EP 4 363 608 B1**
(45) Date of publication and mention of the grant of the patent: **02.04.2025**
(21) Application number: 22741486.9
(22) Date of filing: 30.06.2022
(51) Int. Cl.: C12Q 1/6806, C12Q 1/6841

(54) **UNIT-DNA COMPOSITION FOR SPATIAL BARCODING AND SEQUENCING**
EINHEITS-DNA-ZUSAMMENSETZUNG FÜR RÄUMLICHE BARCODIERUNG UND SEQUENZIERUNG
COMPOSITION D'ADN UNITAIRE POUR LE CODAGE À BARRES ET LE SÉQUENÇAGE SPATIAUX

(30) Priority: 01.07.2021 EP 21183154
(43) Date of publication of application: 08.05.2024
(73) Proprietor: Miltenyi Biotec B.V. & Co. KG, 51429 Bergisch Gladbach (DE)
(72) Inventor: THOMAS, Rothmann, 51429 Bergisch Gladbach (DE); ANDREAS, Bosio, 51429 Bergisch Gladbach (DE); ROBERT, Pinard, 51429 Bergisch Gladbach (DE); MICHEL, Perbost, 51429 Bergisch Gladbach (DE); SANDRA, Halbfeld, 51429 Bergisch Gladbach (DE); MATTHIAS BERNHARD, Wahl, 51429 Bergisch Gladbach (DE)
(74) Representative: Kisters, Michael Marcus
(86) International application number: PCT/EP2022/068213
(87) International publication number: WO 2023/275334

(56) References cited:
- WO-A1-2011/127099
- WO-A1-2017/007925
- WO-A1-2018/050722
- WO-A1-2018/134616
- WO-A1-2019/224544
- WO-A1-2021/116715
- WO-A1-2021/119402
- WO-A1-2021/221831
- WO-A1-2021/257795
- WO-A2-2020/120442
- KISHI JOCELYN Y. ET AL: "Light-Seq: light-directed in situ barcoding of biomolecules in fixed cells and tissues for spatially indexed sequencing", NATURE METHODS, vol. 19, no. 11, 10 October 2022 (2022-10-10), New York, pages 1393 - 1402, XP093077841, ISSN: 1548-7091, Retrieved from the Internet <URL:https://www.nature.com/articles/s41592-022-01604-1> DOI: 10.1038/s41592-022-01604-1

## Description

### BACKGROUND

The invention relates to the technology of spatial sequencing. The aim is to determine the distribution of mRNA in areas of a tissue or in individual cells within the tissue.

Spatial sequencing is a collective term for methods that allow direct sequencing of the mRNA content of a cell in tissue context. These methods can on the one hand serve to analyze mRNA expression profiles of cells in a kind of highly multiplexed fluorescence in situ hybridization (FISH) assay.

On the other hand, in situ sequencing can also enable the read-out of mRNA sequence information, using specific mRNA-binding probes, which can take up a copy of predefined portion of specific mRNA or cDNA sequence ("Gap-fill padlock probes", Ke et al., Nature Methods 2013, doi:10.1038/nmeth.2563). Recently also in situ genome sequencing (IGS) approaches were published (In situ genome sequencing resolves DNA sequence and structure in intact biological samples", A. C. Payne et al., Science 10.1126/science.aay3446 (2020)). All in situ sequencing methods require a signal amplification step, which is in most cases performed by circularization of mRNA- or cDNA-binding probes or gDNA insert circularization by hairpin ligation and subsequent rolling circle amplification (RCA), creating a DNA molecule containing multiple copies of the probe and/or target sequence, the so called Nanoballs, Rolonies or Rolling circle amplification products (RCPs). As these are large molecules with size in nm or µm scale, the number of rolonies that can be formed within one cell is strictly limited by the size of this cell.

Furthermore, if the density of rolonies within cells is too high, discrimination of single mRNA signals during the optical detection step of the sequencing procedure is strongly impaired. As this is a major drawback of the technology, various techniques have been developed to circumvent this, e.g. design of smaller rolonies or generation and clearing of tissue-hydrogel complexes (Asp et al., BioEssays 2020, DOI: 10.1002/bies.201900221) or to expand the cellular target termed expansion sequencing (Alon et al., Science 371, eaax2656 (2021)).

A general process for light-directed in situ barcoding is known from WO2021116715 and WO2021119402 and a general in situ enzymatic polynucleotide synthesis is described in WO2021221831 and WO2019224544.

However, these methods do still not fully evade the inherent spatial limitations of in situ sequencing. Another approach avoids in situ signal amplification: In situ capturing relies on the transfer of mRNA molecules from tissue onto a surface coated with spots of barcoded primers, allowing backtracking of the ex situ gained sequence information to the specific tissue region the sequenced mRNA was extracted from. Nevertheless, this method is also limited, as RNA capture efficiency is restricted and resolution is poor (no single-cell analysis) due to the relatively large size of the barcoded capturing spots (Asp et al., BioEssays 2020, DOI:10.1002/bies.201900221).

### SUMMARY

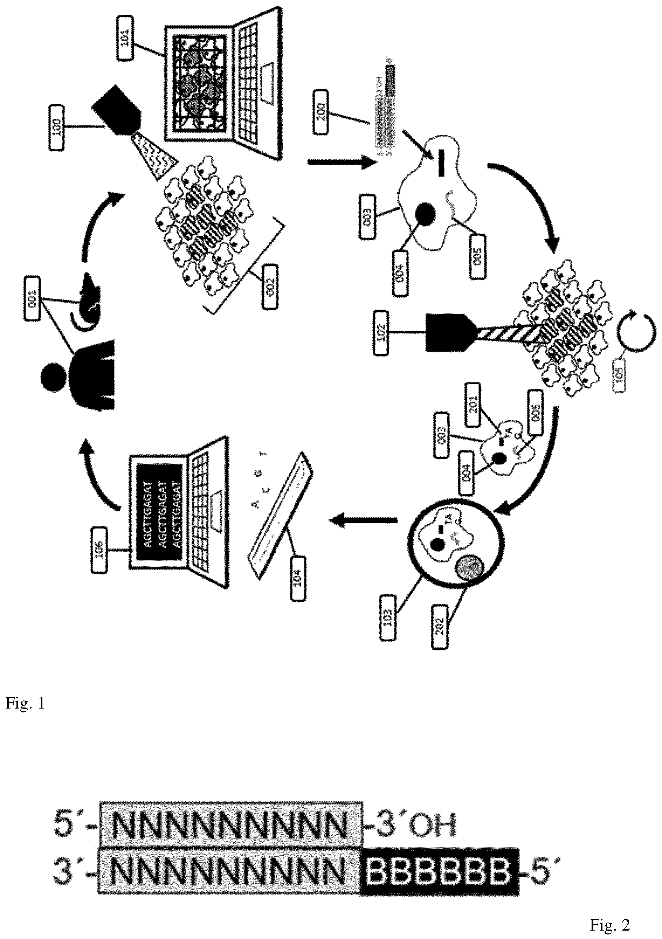

The present invention is directed to a method which uses optical methods to insert a barcode into a polynucleotide, preferable a DNA sequence. This code can be used to retrieve the position at which the coding was carried out. The aim here is that the limitations of existing in situ sequencing methods with regard to the number of measurable mRNA sequences in situ and also the expression dynamics are largely overcome. Optical coding can have a resolution in the range of one µm and a variability of the code that is sufficient for each cell to receive its own code in tissue sections of typical size. The proposed coding and decoding workflow is depicted in Figure 1.

The basic principle as disclosed herein is based on spatial barcoding of nucleic acids by universal template directed DNA synthesis. The method will subsequently be referred to as UNIT-DNA (UNIversal Template DNA).

Object of the invention is therefore a method for in situ spatial barcoding of a polynucleotide comprising a first and a second strand with a barcode nucleotide sequence characterized in that the first strand is provided at its 5' end with an overhang of at least one universal base and the corresponding recessed 3' end of the second strand of the polynucleotide with at least one nucleotide provided with a blocking group, wherein the blocking groups are removed from the incorporated nucleotides by irradiation with light.

Removal of the blocking group may be accomplished by either providing the blocking groups with appropriate photocleavable units or by adding cleaving reagents which are activated or provided in their active form by irradiation with light.

In a first variant of the method, the blocking groups are removed from the incorporated nucleotides by irradiation with light by providing a cleaving reagent, wherein the cleaving reagent is provided by irradiation of a progenitor of the cleaving reagent with light.

In a second variant of the method, wherein the nucleotides are provided with a photocleavable blocking group which is removed from the incorporated nucleotides by irradiation with light. Such reagents are known, for example "Cy5-TECP" which is cleaved by irradiation with light into the active cleaving reagent "Cy5".

In the following, the term "polynucleotide" refers to double stranded nucleic acids, like DNA, RNA, DNA-RNA, c-DNA, ssDNA and similars such as PNA or LNA.

### BRIEF DESCRIPTION OF THE DRAWINGS

Fig. 1 shows the general workflow of the invention including sample preparation, tissue staining and imaging (Transmission, Fluorescence), segmentation or cluster analysis and calculation of masks for the structured illumination and single cell sequencing.
Fig. 2 shows the general structure of a double stranded polynucleotide molecule with at least one 5 overhang with at least one universal base
Fig. 3 shows the basic procedure of barcoding of the invention
Fig. 4: Structured illumination of three cells by light
Fig 5 shows embodiments of the invention depicted A - H
Fig 6 shows the combination of Template Switch Oligonucleotide (TSO) with embodiment H.
Fig. 7 shows the general workflow of embodiment H of the invention with in vitro sequencing by tagging molecules directly used for sequencing.
Fig. 8 shows the combination of circular ssDNA with UNIT-DNA embodiment H
Fig. 9 shows the combination of targeted DNA amplification with UNIT-DNA embodiment H

### DETAILED DESCRIPTION

The proposed UNIT-DNA workflow is depicted in Fig. 1 for the coding workflow according to embodiment A. Sample Preparation, tissue staining and imaging (Transmission, Fluorescence) is followed by segmentation or cluster analysis and calculation of masks for the structured illumination. For the decoding workflow depicted in Fig. 1 with UNIT-DNA, a tissue section is provided with spatial barcoding of cells, guided by structured illumination and cyclic incorporation of nucleotides (fixation of coded UNIT-DNA to tissue before cell release is not shown). Release of cells from tissue section and encapsulation of cells for single cell sequencing.

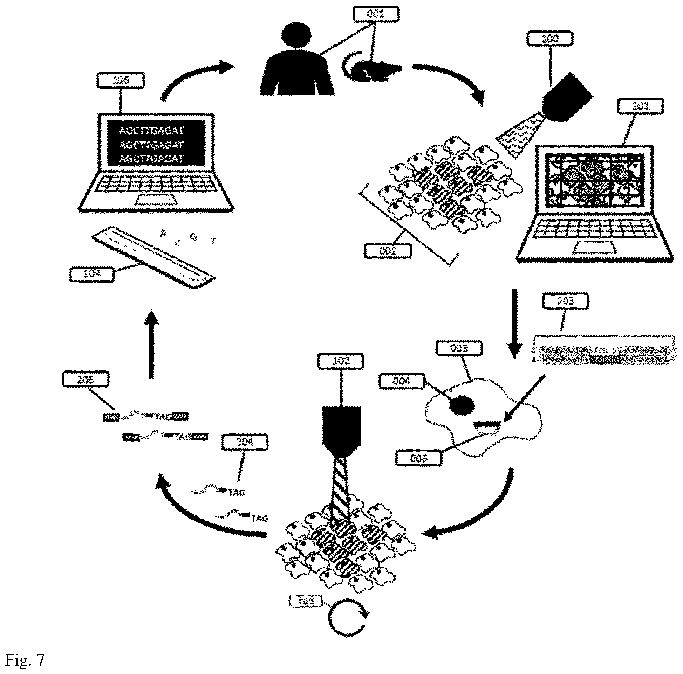

For the decoding workflow depicted in Fig 7 according to embodiment H with UNIT-DNA, a tissue section is provided with spatial barcoding of target molecules guided by structured illumination and cyclic incorporation of nucleotides. The sequence obtained for the target mRNA, as well as the spatial barcode are annotated to images. Bioinformatics analysis and relation of the results to initial sample source complete the workflow.

The method of UNIT-DNA consists of providing a double stranded DNA molecule comprising a first and a second strand with a barcode nucleotide sequence with at least one 5 overhang, where the 5' overhang includes at least one universal base and the recessed 3' end has a free 3'-OH.

Fig. 2 shows an example of a composition obtained by the method of the invention for a 9 bp double stranded nucleic acid (N) and for a 6 universal base (B) 5'overhang. The number of bp for the double stranded nucleic acid or the number of universal bases for single stranded 5' overhang may vary in length.

The term "universal base" refers to nucleotides which are able to bind to all natural nucleotides. Such universal base designs have been described in the literature mainly as part of degenerate primers or probes due to their property to pair with all natural bases (e.g by Loakes, Nucleic Acid Research, 2001, Vol. 29, No. 12 2437-2447).

The UNIT-DNA composition obtained by the method of the invention is shown in Fig 2 and consists of a double stranded nucleic acid with at least one 5 overhang, where the 5' overhang includes at least one universal base and the recessed 3' end has a free 3'-OH. Here as an example the UNIT-DNA composition for a 9 bp double stranded nucleic acid and for a 5 universal base 5 overhang is shown. N stands for natural base (G or C or T or A) and B stands for universal base.

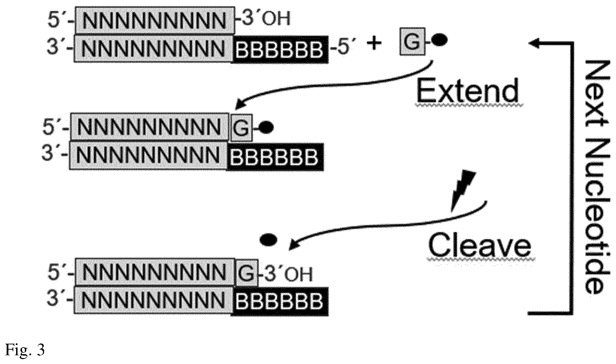

Fig. 3 shows the basic principle of coding by the UNIT-DNA method. With the support of a polymerase (not shown) the recessed free 3'-OH of the second strand is incorporating the nucleotide provided (G). Thereby the recessed 3'-OH second strand is extended and coded by the first nucleotide. Any nucleotide provided will pair with the composition as long as the 5' overhang of the first strand is including universal bases which support the universal template directed DNA synthesis. The recessed 3'-OH is only extended by one nucleotide as the 3' end of the nucleotide is blocked. As a next step, the blocked 3'-OH is unblocked by a cleave reagent which allows the next coding cycle to occur.

The incorporation of an optionally fluorescently labeled 3'-OH blocked nucleotides which are later unblocked by a cleave reagent is also known from Sequencing by Synthesis (Chen et al., Genomics, Proteomics & Bioinformatics, Volume 11, Issue 1, February 2013, Pages 34-40). Opposite to Sequencing by Synthesis, the UNIT-DNA process is used to write a DNA code and not to read a DNA code.

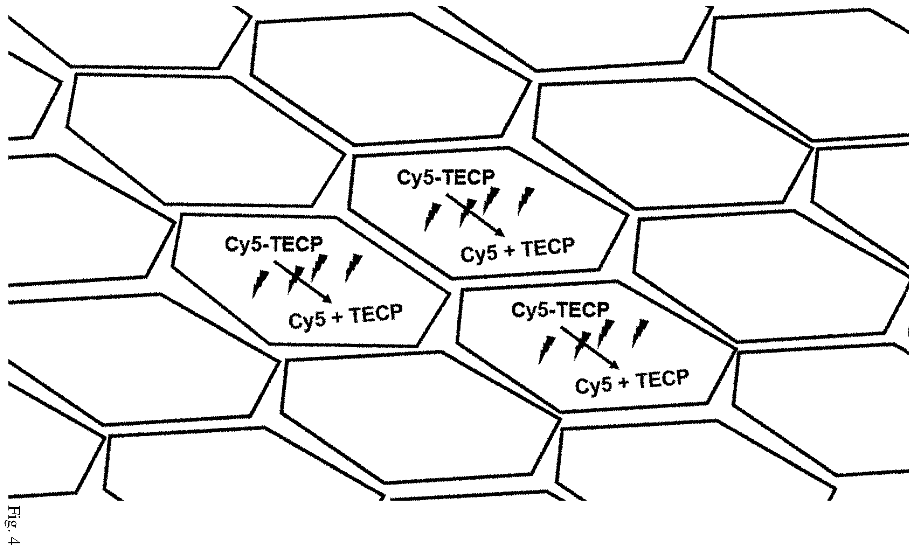

In order to write the spatial polynucleotide barcode, structured illumination may be used as part of the coding workflow which was already conceptually introduced by Fig, 1. How the structured illumination of individual cells by light (e.G. UV light) is chemically releasing the cleave reagent (e.G. TCEP) spatially is detailed in Fig. 4. The chemical reaction to release TCEP after illumination of Cy5-TCEP conjugate by UV light has been described before (Vaughan et al., J Am Chem Soc., 2013 Jan 30, 135(4), 1197-1200).

In summary, the sequence of the spatial code written by the UNIT-DNA method depends on the order of the nucleotides provided and the spatial activation of the cleave reagent by light. The total number of spatial codes which can be written by UNIT-DNA depends on the number universal bases within the 5'overhang which allow nucleotide incorporation (e.G. 10 universal bases would translate to ~1 million codes (4¹⁰)). The spatial resolution of the coding principle is dependent on the resolution of light used for illumination (~300nm for UVB) and the local reaction kinetics of the released cleave reagent and is therefore easily achieving a cellular (~10µm) or subcellular (~1µm) resolution level.

After the coding has been completed, all cells (or nuclei and organells) may be isolated from the tissue sample and are subjected to single cell sequencing. In principle, the method is not limited in terms of the number of cells examined simultaneously. The number of cells examined individually at the same time is dependent on the number of universal bases within the 5 overhang to provide a unique spatial barcode. The real limitation is eventually only in the capacity and throughput of the sequencer.

### Embodiments of UNIT-DNA spatial barcoding

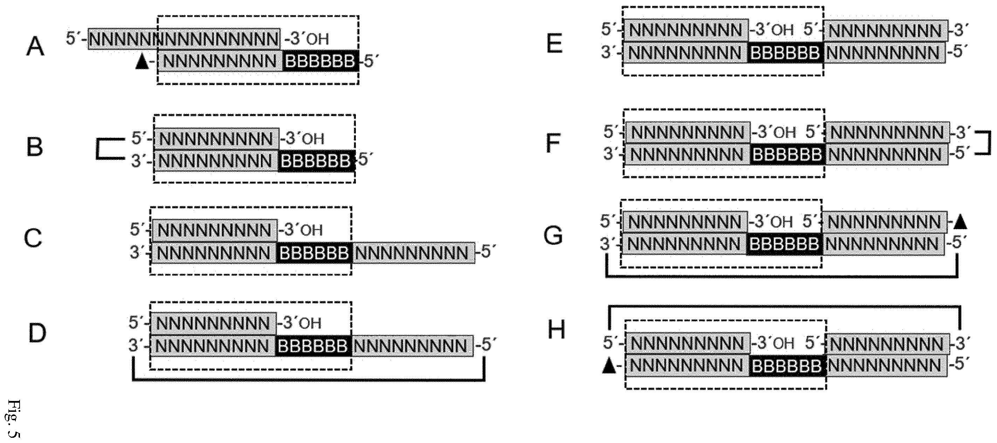

The embodiments of the method of the invention for spatial barcoding are summarized in Fig. 5 and referred to as embodiments A to H. As visualized by the dotted box, the core functional elements of the UNIT-DNA method is maintained. Additional functionality is introduced by modifications of the 3' and 5 ends and combines the core UNIT-DNA composition for spatial barcoding with further nucleic acid manipulation workflows.

The embodiments are described in more detail as follows

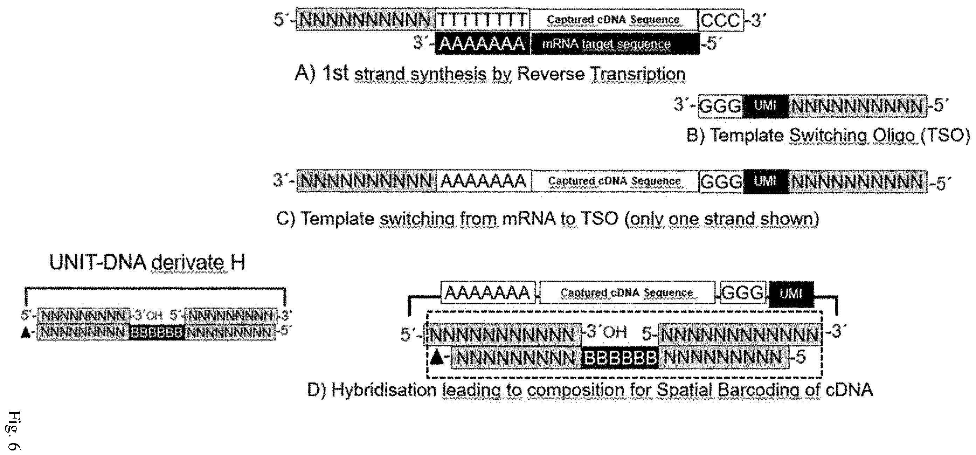

Fig. 6 shows an example for UNIT-DNA embodiment H (Fig. 5) which is used as part of the template switch oligonucleotide process within the single cell sequencing workflow (Picelli et al., Nat Methods 2013 Nov;10(11):1096-8. doi: 10.1038/nmeth.2639. Epub 2013 Sep 22)). After generation of the spatial DNA barcode by the UNIT-DNA, the resulting nucleic acid can be further analyzed by sequencing using the unique molecular identifier (UMI) for error correction.

It is worth to mention that the TSO shown in Fig. 6 does not include a Cell Identifier. The UNIT-DNA composition provides the spatial barcode which can serve as a cell identifier in case resolution of structured illumination was chosen to be aligned with the cellular resolution level.

Fig. 7 is updating the coding and decoding workflow for use of the UNIT-DNA derivate in embodiment H. After coding a sequencing library is prepared and the spatial barcode as well as the target nucleic acid is sequenced. As the spatial barcode is physically linked to the target nucleic acid, the spatial information of the target sequence can be derived by in vitro sequencing and the relation of the results to the initial sample source is provided.

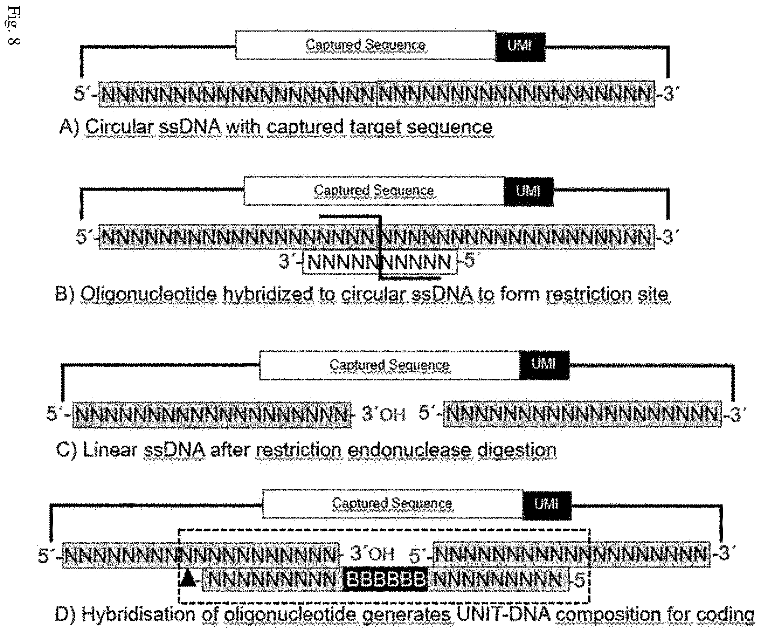

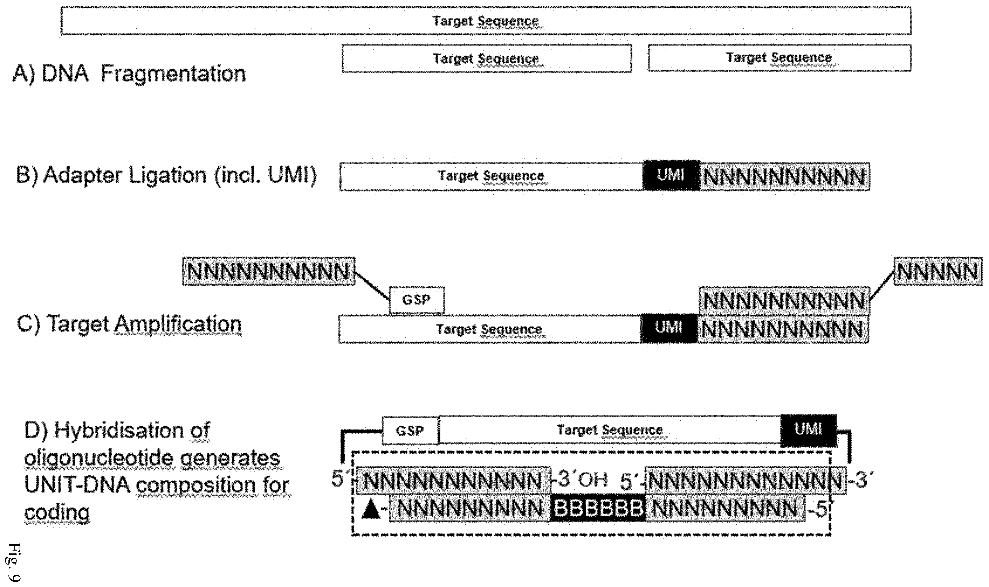

The UNIT-DNA composition H for spatial barcoding of the target nucleic acid can also be used within a padlock workflow leading to a circularized ssDNA (see Figure 8) or within a targeted DNA workflow (see Fig. 9). After coding, the resulting nucleic acid would be subjected to sequencing in order to determine the spatial barcode and the linked target nucleic acid.

Depending on the molecular workflow different UNIT-DNA embodiments may be used to combine the spatial coding with the sequencing and decoding workflow. The UNIT-DNA embodiments as shown in Fig. 5 may also be used for multimodal targeted RNA and DNA workflows or solid support workflows (not shown).

The UNIT-DNA process may be performed within a cyclic process, which is triggered by a structured illumination of the tissue sample by treating it with another spatially structured pattern of light in each cycle. "Structured illumination" and "spatially structured pattern of light" refer to illuminating only a part or selected areas of the sample.

Fig. 4 shows structured illumination of three cells by light (as indicated by the thunder symbol) is releasing the cleave reagent TCEP (tris(2-carboxyethyl)phosphine) in the illuminated cells. Cy5-TECP conjugate is used as a substrate for the light induced cleave reagent release.

Further, in Fig. 1 it is shown how a tissue section 002 (optionally stained) is obtained from tissue donor 001 which is then subjected to imaging 100, allowing segmentation or cluster analysis, i.e. the selection of the parts of the sample to be further investigated by the method of the invention. Such segmentation/clustering/selection enables calculation of masks for the structured illumination and/or for spatially structured pattern of light.

Further downstream in the method of the invention, the information obtained for structured illumination and/or for spatially structured pattern of light is utilized during photo-treatment for UNIT-DNA code generation (102) which leads to the encoded UNIT-DNA which is encapsulated together with the cellular mRNA and the single cell indexing reagents (202) for later sequencing (104).. With the aid of the structured illumination, only the selected areas/cells of the sample 106 which are provided with the spatial barcode are spatially decoded by Next generation sequencing (104) and Sequence analysis (106).

### Sequencing

One step in the method of the invention is directed to determine the sequence of nucleotides encoded on the UNIT-DNA probes which is read out by sequencing (104). One method for sequencing can be sequencing be synthesis (SBS). For increasing readout signals, amplification of the UNIT-DNA probe sequences can be performed. One method for clonal amplification can be rolling circle amplification (RCA) of the encoded UNIT-DNA probes, which is performed before starting the sequencing process on the rolonies.

In a variant of the invention, the sequence of the UNIT-DNA code may be read separately from the sequence of the target gene. This can be realized by splitting up the sequencing procedure into two runs with two different sequencing primers.

### Embodiments of the invention

Eight embodiments (A - H) of the UNIT-DNA method are shown in Fig. 5. The core elements of the UNIT-DNA composition are maintained for all embodiments (as indicated by the dotted box). The additional elements added to the 5' and 3 end of the UNIT-DNA are as follows.
(A) A second 5'overhang with a blocked 3' end within the overhang. In embodiments A and B, the first strand is further provided at its 3'-end with a blocking group
(B) The 5 end of the double strand is linked to the 3 end of the double strand.
(C) The 5' end of the 5' universal base overhang is extended by natural bases. In embodiment C, the overhang of the first strand is provided at its 5' -end with a first oligonucleotide.
(D) The 5' end of the 5' universal base overhang extended by natural bases is linked to the 3 end of the double strand. In embodiment D, the first oligonucleotide is ligated directly or via an oligonucleotide bridge to the 3' end of the first strand thereby forming a circle. The oligonucleotide bridge may have a length of 5 to 100 nucleotides.
(E) The 5' end of the 5' universal base overhang extended by natural bases is forming a double strand of natural baes. In embodiment E, the first oligonucleotide is hybridized with the corresponding nucleotides thereby obtaining a polynucleotide having blunt ends and a gap at the location of the at least one universal base.
(F) The 5' end of the 5' universal base overhang extended by natural bases forming a double strand of natural bases is linked with the 3 end of the neighboring double strand.
   In embodiment F, the first oligonucleotide is hybridized with the corresponding nucleotides thereby obtaining a polynucleotide having blunt ends and a gap at the location of the at least one universal base and wherein the blunt ends are ligated with each other directly or via an oligonucleotide bridge. The oligonucleotide bridge may have a length of 5 to 100 nucleotides.
(G) The 5' end of the 5' universal base overhang extended by natural bases forming a double strand of natural bases with a blocked 3 end while the 5 end is linked with the opposite 3 end of the double strand forming a circle. In embodiment G, the first oligonucleotide is hybridized with the corresponding nucleotides thereby obtaining a polynucleotide having blunt ends and a gap at the location of the at least one universal base and wherein the first oligonucleotide is ligated directly or via an oligonucleotide bridge to the 3' end of the first strand thereby forming a circle and the 3'-end of the hybridized corresponding nucleotides contain a non-cleavable blocking group. The oligonucleotide bridge may have a length of 5 to 100 nucleotides.
(H) The 5' end of the double strand is linked to the 3' end of the opposite double strand forming a padlock like structure with the 3 end of the double strand blocked. In embodiment G, the first oligonucleotide is hybridized with the corresponding nucleotides thereby obtaining a polynucleotide having blunt ends and a gap at the location of the at least one universal base and wherein the 3' end of the hybridized corresponding nucleotides is ligated directly or via an oligonucleotide bridge to the 5' end of the second strand thereby forming a circle and the 3'-end of the first strand contains a non-cleavable blocking group. The oligonucleotide bridge may have a length of 5 to 100 nucleotides.

Embodiment H may be conducted in a first variant as shown in Fig. 6. Here, the UNIT-DNA method of the invention is combined with a Template Switch Oligonucleotide (TSO) process.

The variant as shown in Fig. 6 comprises that (A) mRNA is reverse transcribed (in situ) by oligo dT primer generating cDNA with triple C at the 3' end. (B) TSO with 5' PCR handle (shown as N), Unique Molecular Identifier (UMI) and triple G at 3 end. (C) Result of in situ template switching of cDNA (from A) with TSO (from B) generates captured cDNA with PCR handle at both ends (optional in situ PCR amplification for increased sensitivity not shown). (D) Hybridization of oligonucleotide which includes universal bases with PCR handle is leading to formation of UNIT-DNA derivate H (as indicated by the dotted box).

A further variant of embodiment H is shown in Fig. 8. Here, the UNIT-DNA method of the invention is combined with a circular ssDNA.

The variant as shown in Fig. 8 comprises (A) Circular ssDNA (including captured target sequence, unique molecular identifier (UMI) and PCR handle (shown as N)). UMIs are added before amplification and are often used within NGS workflows to reduce errors and quantitative bias introduced by the amplification. The Circular ssDNA may have been generated in situ by Padlock (no gap), Padlock (gap) or Direct (FISSEQ) method as described by Chen et al. (Nucleic Acids Research, 2018, Vol. 46, No. 4 e22). (B) Oligo hybridization will generate a double strand and allows restriction endonuclease digestion as shown under (B) to generate a linear ssDNA (C) (optional in situ PCR amplification for increased sensitivity not shown). (D) Hybridization of oligonucleotide which includes universal bases with PCR handle is leading to formation of UNIT-DNA derivate H (as indicated by the dotted box).

A further variant of embodiment H is shown in Fig. 9. Here, the UNIT-DNA method of the invention is combined with a targeted DNA amplification, for example by the Targeted DNA workflow by QIAGEN as disclosed in the QIAseq Targeted DNA Panel Handbook 03/2021, Figure 2) was modified to combine with UNIT-DNA derivate H for spatial (B). The Adapter includes a unique molecular identifier (UMI) and PCR handle (N). (C) For enrichment the ligated target sequence is subjected to several cycles of in situ targeted PCR by a Gene Specific Primer (GSP with PCR handle) and a generic primer (shown as N). (D) Hybridization of oligonucleotide which includes universal bases with PCR handle is leading to formation of UNIT-DNA derivate H (as indicated by the dotted box).

### Glossary for figs 1 and 7

- 001: Tissue donor
- 002: Stained tissue section
- 003: Cell
- 004: Cell nucleus
- 005: mRNA in cytoplasm
- 006: mRNA (linked to UNIT-DNA composition)
- 100: Imaging
- 101: Segmentation or cluster analysis, calculation of masks for the structured illumination
- 102: Photo-treatment for UNIT-DNA Code generation
- 103: Single Cell Encapsulation
- 104: Sequencing
- 105: Cyclic barcoding
- 106: Sequence analysis
- 200: UNIT-DNA composition before coding
- 201: UNIT-DNA composition after coding
- 202: Single Cell Indexing reagents
- 203: UNIT-DNA composition H
- 204: Linearized Template switched cDNA with spatial barcode
- 205: Sequencing Library derived from cDNA

## Claims

1. Method for in situ spatial barcoding of a polynucleotide comprising a first and a second strand with a barcode nucleotide sequence **characterized in that** the first strand is provided at its 5' end with an overhang of at least one universal base and the corresponding recessed 3' end of the second strand of the polynucleotide with at least one nucleotide provided with a blocking group, wherein the blocking groups are removed from the incorporated nucleotides by irradiation with light.

2. Method according to claim1 **characterized in that** the blocking groups are removed from the incorporated nucleotides by irradiation with light by providing a cleaving reagent, wherein the cleaving reagent is provided by irradiation of a progenitor of the cleaving reagent with light.

3. Method according to claim1 **characterized in that** wherein the nucleotides are provided with a photocleavable blocking group which is removed from the incorporated nucleotides by irradiation with light.

4. Method according to any of the claims 1 to 3 **characterized in that** the first strand is further provided at its 3'-end with a blocking group.

5. Method according to any of the claims 1 to 4 **characterized in that** the overhang of the first strand is provided at its 5'-end with a first oligonucleotide.

6. Method according to claim 5 **characterized in that** the first oligonucleotide is ligated directly or via an oligonucleotide bridge to the 3' end of the first strand thereby forming a circle.

7. Method according to claim 5 **characterized in that** the first oligonucleotide is hybridized with the corresponding nucleotides thereby obtaining a polynucleotide strand having blunt ends and a gap at the location of the at least one universal base.

8. Method according to claim 5 **characterized in that** the first oligonucleotide is hybridized with the corresponding nucleotides thereby obtaining a polynucleotide having blunt ends and a gap at the location of the at least one universal base and wherein the blunt ends are ligated with each other directly or via an oligonucleotide bridge.

9. Method according to claim 5 **characterized in that** the first oligonucleotide is hybridized with the corresponding nucleotides thereby obtaining a polynucleotide having blunt ends and a gap at the location of the at least one universal base and wherein the first oligonucleotide is ligated directly or via an oligonucleotide bridge to the 3' end of the first strand thereby forming a circle and the 3'-end of the hybridized corresponding nucleotides contain a non-cleavable blocking group.

10. Method according to claim 5 **characterized in that** the first oligonucleotide is hybridized with the corresponding nucleotides thereby obtaining a polynucleotide strand having blunt ends and a gap at the location of the at least one universal base and wherein the 3' end of the hybridized corresponding nucleotides is ligated directly or via an oligonucleotide bridge to the 5' end of the second strand thereby forming a circle and the 3'-end of the first strand contains a non-cleavable blocking group.

11. Method according to any of the claims 1 to 10 **characterized in that** the polynucleotide strand is provided by Template switching of an m-RNA strand as a result of steps: a) 1^{st} strand synthesis by reverse transcription of mRNA by oligo dT priming leading to C nucleotide at the 3' end added to the captured target sequences followed by b) hybridization of the template switching oligo by corresponding G nucleotides at the 3 end resulting into the template switched cDNA which is c) hybridizing to the corresponding first strand nucleotides generating a free 3'OH and a gap at the location of the at least one universal base.

12. Method according to any of the claims 1 to 10 **characterized in that** the DNA strand is provided by padlock workflow leading circular ssDNA template as a result of steps: a) Circular ssDNA with captured target sequence as a result of padlock probe hybridization (including gap fill reaction for gap fill padlock probes) and ligation. b) Oligonucleotide hybridization to circular ssDNA to allow dsDNA restriction resulting into linear ssDNA which is C) hybridizing to the corresponding first strand nucleotides generating a free 3'OH and a gap at the location of the at least one universal base.

13. Method according to any of the claims 1 to 10 **characterized in that** the DNA strand is provided by targeted DNA amplification as a result of steps: a) DNA fragmentation and adapter ligation. b) Enrichment of the ligated target sequence by PCR with a gene specific primer (with PCR handle) and a generic primer resulting into linear ssDNA which is C) hybridizing to the corresponding first strand nucleotides generating a free 3'OH and a gap at the location of the at least one universal base.

## Patentansprüche

1. Verfahren zum räumlichen In-situ-Barcoding eines Polynukleotids, das einen ersten und einen zweiten Strang mit einer Barcode-Nukleotidsequenz umfasst, **dadurch gekennzeichnet, dass** der erste Strang an seinem 5'-Ende mit einem Überhang von mindestens einer universellen Base und das entsprechende rezessive 3'-Ende des zweiten Strangs des Polynukleotids mit mindestens einem Nukleotid bereitgestellt wird, das mit einer blockierenden Gruppe bereitgestellt wird, wobei die blockierenden Gruppen durch Bestrahlung mit Licht von den eingebauten Nukleotiden entfernt werden.

2. Verfahren nach Anspruch 1, **dadurch gekennzeichnet, dass** die blockierenden Gruppen von den eingebauten Nukleotiden durch Bestrahlung mit Licht durch Bereitstellen eines Spaltreagenz entfernt werden, wobei das Spaltreagenz durch Bestrahlung eines Vorläufers des Spaltreagenzes mit Licht bereitgestellt wird.

3. Verfahren nach Anspruch 1, **dadurch gekennzeichnet, dass** die Nukleotide mit einer fotospaltbaren blockierenden Gruppe bereitgestellt werden, die durch Bestrahlung mit Licht von den eingebauten Nukleotiden entfernt wird.

4. Verfahren nach einem der Ansprüche 1 bis 3, **dadurch gekennzeichnet, dass** der erste Strang an seinem 3'-Ende ferner mit einer blockierenden Gruppe bereitgestellt wird.

5. Verfahren nach einem der Ansprüche 1 bis 4, **dadurch gekennzeichnet, dass** der Überhang des ersten Stranges an seinem 5'-Ende mit einem ersten Oligonukleotid bereitgestellt wird.

6. Verfahren nach Anspruch 5, **dadurch gekennzeichnet, dass** das erste Oligonukleotid direkt oder über eine Oligonukleotidbrücke an das 3'-Ende des ersten Strangs ligiert wird, wodurch ein Kreis gebildet wird.

7. Verfahren nach Anspruch 5, **dadurch gekennzeichnet, dass** das erste Oligonukleotid mit den entsprechenden Nukleotiden hybridisiert wird, wodurch ein Polynukleotidstrang mit stumpfen Enden und einer Lücke an der Stelle der mindestens einen universellen Base erhalten wird.

8. Verfahren nach Anspruch 5, **dadurch gekennzeichnet, dass** das erste Oligonukleotid mit den entsprechenden Nukleotiden hybridisiert wird, wodurch ein Polynukleotidstrang mit stumpfen Enden und einer Lücke an der Stelle der mindestens einen universellen Base erhalten wird, und wobei die stumpfen Enden direkt oder über eine Oligonukleotidbrücke miteinander ligiert werden.

9. Verfahren nach Anspruch 5, **dadurch gekennzeichnet, dass** das erste Oligonukleotid mit den entsprechenden Nukleotiden hybridisiert wird, wodurch ein Polynukleotid mit stumpfen Enden und einer Lücke an der Stelle der mindestens einen universellen Base erhalten wird, und wobei das erste Oligonukleotid direkt oder über eine Oligonukleotidbrücke an das 3'-Ende des ersten Strangs ligiert wird, wodurch ein Kreis gebildet wird, und das 3'-Ende der hybridisierten entsprechenden Nukleotide eine nicht spaltbare blockierende Gruppe enthält.

10. Verfahren nach Anspruch 5, **dadurch gekennzeichnet, dass** das erste Oligonukleotid mit den entsprechenden Nukleotiden hybridisiert wird, wodurch ein Polynukleotidstrang mit stumpfen Enden und einer Lücke an der Stelle der mindestens einen universellen Base erhalten wird, und wobei das 3'-Ende der hybridisierten entsprechenden Nukleotide direkt oder über eine Oligonukleotidbrücke an das 5'-Ende des zweiten Strangs ligiert wird, wodurch ein Kreis gebildet wird, und das 3'-Ende des ersten Strangs eine nicht spaltbare blockierende Gruppe enthält.

11. Verfahren nach einem der Ansprüche 1 bis 10, **dadurch gekennzeichnet, dass** der Polynukleotidstrang durch Template-Switching eines m-RNA-Strangs als Ergebnis der folgenden Schritte bereitgestellt wird: a) Synthese eines ersten Stranges durch reverse Transkription von mRNA mittels Oligo-dT-Priming, das zu einem C-Nukleotid am 3'-Ende führt, das an die eingefangenen Zielsequenzen angehängt wird, gefolgt von b) Hybridisierung des Template-Switching-Oligos mit entsprechenden G-Nukleotiden am 3'-Ende, was zu einer template-switched cDNA führt, die c) mit den entsprechenden Nukleotiden des ersten Stranges hybridisiert und ein freies 3'OH und eine Lücke an der Stelle der mindestens einen universellen Base erzeugt.

12. Verfahren nach einem der Ansprüche 1 bis 10, **dadurch gekennzeichnet, dass** der DNA-Strang durch einen Padlock-Workflow, der zu einem zirkulären ssDNA-Template führt, als Ergebnis der folgenden Schritte bereitgestellt wird: a) Zirkuläre ssDNA mit eingefangener Zielsequenz als Ergebnis der Padlock-Sonden-Hybridisierung (einschließlich der Lückenfüllreaktion für lückenfüllende Padlock-Sonden) und Ligation, b) Oligonukleotid-Hybridisierung an zirkuläre ssDNA, um dsDNA-Restriktion zu ermöglichen, was zu linearer ssDNA führt, die c) an die entsprechenden Nukleotide des ersten Strangs hybridisiert und ein freies 3'OH und eine Lücke an der Stelle der mindestens einen universellen Base erzeugt.

13. Verfahren nach einem der Ansprüche 1 bis 10, **dadurch gekennzeichnet, dass** der DNA-Strang durch gezielte DNA-Amplifikation als Ergebnis der folgenden Schritte bereitgestellt wird: a) DNA-Fragmentierung und Adapter-Ligation, b) Anreicherung der ligierten Zielsequenz durch PCR mit einem genspezifischen Primer (mit PCR-Handle) und einem generischen Primer, was zu linearer ssDNA führt, die c) mit den entsprechenden Nukleotiden des ersten Strangs hybridisiert und ein freies 3'OH und eine Lücke an der Stelle der mindestens einen universellen Base erzeugt.

## Revendications

1. Procédé pour le codage à barres spatial in situ d'un polynucléotide comprenant un première et un second brins avec une séquence nucléotidique de code-barre **caractérisé en ce que** le premier brin est fourni à son extrémité 5' avec une saillie d'au moins une base universelle et l'extrémité 3' en retrait correspondante du second brin du polynucléotide avec au moins un nucléotide fourni avec un groupe de blocage, les groupes de blocage étant enlevés des nucléotides incorporés par irradiation avec de la lumière.

2. Procédé selon la revendication 1, **caractérisé en ce que** les groupes de blocage sont enlevés des nucléotides incorporés par irradiation avec de la lumière en fournissant un réactif de clivage, le réactif de clivage étant fourni par irradiation d'un progéniteur du réactif de clivage avec de la lumière.

3. Procédé selon la revendication 1, **caractérisé en ce que** dans lequel les nucléotides sont fournis avec un groupe de blocage photoclivable qui est enlevé des nucléotides incorporés par irradiation avec de la lumière.

4. Procédé selon l'une quelconque des revendications 1 à 3, **caractérisé en ce que** le premier brin est en outre fourni à son extrémité 3' avec un groupe de blocage.

5. Procédé selon l'une quelconque des revendications 1 à 4, **caractérisé en ce que** la saillie du premier brin est fournie à son extrémité 5' avec un premier oligonucléotide.

6. Procédé selon la revendication 5, **caractérisé en ce que** le premier oligonucléotide est ligué directement ou via un pont d'oligonucléotide à l'extrémité 3' du premier brin formant ainsi un cercle.

7. Procédé selon la revendication 5, **caractérisé en ce que** le premier oligonucléotide est hybridé avec les nucléotides correspondants obtenant ainsi un brin de polynucléotide ayant des bouts francs et un espace libre au site de l'au moins une base universelle.

8. Procédé selon la revendication 5, **caractérisé en ce que** le premier oligonucléotide est hybridé avec les nucléotides correspondants obtenant ainsi un polynucléotide ayant des bouts francs et un espace libre au site de l'au moins une base universelle et les bouts francs étant ligués l'un avec l'autre directement ou via un pont d'oligonucléotide.

9. Procédé selon la revendication 5, **caractérisé en ce que** le premier oligonucléotide est hybridé avec les nucléotides correspondants obtenant ainsi un brin de polynucléotide ayant des bouts francs et un espace libre au site de l'au moins une base universelle et le premier oligonucléotide étant ligué directement ou via un pont d'oligonucléotide à l'extrémité 3' du premier brin formant ainsi un cercle et l'extrémité 3' des nucléotides correspondants hybridés contenant un groupe de blocage non clivable.

10. Procédé selon la revendication 5, **caractérisé en ce que** le premier oligonucléotide est hybridé avec les nucléotides correspondants obtenant ainsi un brin de polynucléotide ayant des bouts francs et un espace libre au site de l'au moins une base universelle et l'extrémité 3' des nucléotides correspondants hybridés étant liguée directement ou via un pont d'oligonucléotide à l'extrémité 5' du second brin formant ainsi un cercle et l'extrémité 3' du premier brin contenant un groupe de blocage non clivable.

11. Procédé selon l'une quelconque des revendications 1 à 10, **caractérisé en ce que** le brin de polynucléotide est fourni par commutation de matrice d'un brin d'ARNm en résultat des étapes : a) synthèse de 1^{er} brin par transcription inverse de l'ARNm par amorçage d'oligo dT conduisant au nucléotide C à l'extrémité 3' ajouté aux séquences cibles capturées suivi par b) l'hybridation de l'oligo de commutation de matrice par les nucléotides G correspondants à l'extrémité 3' résultant dans l'ADNc commuté de matrice qui est c) s'hybridant aux nucléotides du premier brin correspondants produisant un 3' OH libre et un espace libre au site de l'au moins une base universelle.

12. Procédé selon l'une quelconque des revendications 1 à 10, **caractérisé en ce que** le brin d'ADN est fourni par processus de verrou conduisant la matrice d'ADNsb circulaire en résultat des étapes : a) l'ADNsb circulaire avec la séquence cible capturée en résultat de l'hybridation de sonde de verrou (comprenant la réaction de remplissage d'espace libre pour les sondes de verrou de remplissage d'espace libre) et une ligation. b) l'hybridation d'oligonucléotide à l'ADNsb circulaire pour permettre la restriction d'ADNdb en ADNsb linéaire qui est c) s'hybridant aux nucléotides de premier brin correspondants produisant un 3' OH libre et un espace libre au site de l'au moins une base universelle.

13. Procédé selon l'une quelconque des revendications 1 à 10, **caractérisé en ce que** le brin d'ADN est fourni par amplification d'ADN ciblé en résultat des étapes : a) fragmentation d'ADN et ligation d'un adaptateur. b) enrichissement de la séquence cible liguée par PCR avec une amorce spécifique d'un gène (avec manipulation de PCR) et une amorce générique résultant dans un ADNsb linéaire qui est c) s'hybridant aux nucléotides de premier brin correspondants produisant un 3' OH libre et un espace libre au site de l'au moins une base universelle.
